# EUROPÄISCHE PATENTSCHRIFT

(11) **EP 3 217 916 B1**
(45) Veröffentlichungstag und Bekanntmachung des Hinweises auf die Patenterteilung: **21.10.2020**
(21) Anmeldenummer: 16837998.0
(22) Anmeldetag: 22.12.2016
(51) Int. Cl.: A61B 90/00

(54) **FORMBARES ODER STARRES GEBILDE ZUR MARKIERUNG EINES ORTS ODER BEREICHS IM ODER AM MENSCHLICHEN ODER TIERISCHEN KÖRPER**
SHAPEABLE OR RIGID STRUCTURE FOR MARKING A LOCATION OR A REGION IN OR ON THE HUMAN OR ANIMAL BODY
STRUCTURE DÉFORMABLE OU RIGIDE DE MARQUAGE D'UN SITE OU D'UNE ZONE DANS LE CORPS HUMAIN OU ANIMAL, OU SUR CE DERNIER

(30) Priorität: 29.01.2016 DE 102016201421
(43) Veröffentlichungstag der Anmeldung: 20.09.2017
(73) Patentinhaber: SurgMark GmbH, 22393 Hamburg (DE)
(72) Erfinder: SCHURE Viola, 16359 Biesenthal (DE); SCHURE Frank, 16359 Biesenthal (DE)
(74) Vertreter: Heinemeyer, Karsten
(86) Internationale Anmeldenummer: PCT/DE2016/200576
(87) Internationale Veröffentlichungsnummer: WO 2017/129162

(56) Entgegenhaltungen:
- EP-A1- 1 163 888
- WO-A1-01/08578
- WO-A2-2014/149183
- US-A1- 2005 038 355
- US-B1- 8 798 716

## Beschreibung

Die Erfindung betrifft ein formbares oder starres Gebilde zur Markierung eines Orts oder Bereichs im oder am menschlichen oder tierischen Körper.

Zur Markierung eines Orts oder Bereichs im oder am menschlichen oder tierischen Körper sind unterschiedliche Einrichtungen oder Gebilde bekannt. Dabei werden insbesondere Clipmarkierungen, Drahtmarkierungen oder Fadenmarkierungen verwendet. Hierbei ist von Bedeutung, dass diese Einrichtungen oder Gebilde bei bildgebenden Verfahren oder auch direkt visuell oder bei endoskopischen Verfahren erkennbar sind. Solche Markierungen sollen je nach Anwendungsfall auch nach längeren Zeiträumen, beispielsweise bei erneuten Operationen, wieder auffindbar sein.

Die bekannten Markierungen weisen individuelle Vorteile und Nachteile je nach Anwendungsfall auf. Beispielsweise stören in eine Brust eingebrachte Clipmarkierungen beim Tasten und verursachen hierbei häufig nadelstichartige Schmerzen. Derartige Clipmarkierungen sind bei Röntgenverfahren, CT-Verfahren und MRT-Verfahren gut sichtbar, wohingegen sie bei Ultraschall-Verfahren nur in den seltensten Fällen auffindbar sind. Des Weiteren sind Clipmarkierungen bei Operationen nur mit "Durchleuchtung" (Röntgenbildwandler) sichtbar, wobei sie weiterhin eine Präparatradiografie erfordern.

Drahtmarkierungen verschieben sich nach deren Einbringen in den Körper häufig beim Transport des Patienten oder bei Bewegung des Markierungsorts. Des Weiteren sind Drahtmarkierungen bei der Operation nicht dreidimensional darstellbar und erfordern ebenfalls eine Präparatradiografie.

Herkömmliche Fadenmarkierungen sind durch bildgebende Verfahren nicht darstellbar. Es erfordert daher intraoperativ viel Mühe, derartige Fäden im Körper wieder aufzufinden. EP-A-1163888 und WO-A-01/08578 zeigen jeweils Markierungsvorrichtungen für Weichgewebe. US-B-8798716 zeigt einen Marker für die nicht-invasive Anwendung.

Der vorliegenden Erfindung liegt nunmehr die Aufgabe zugrunde, ein Gebilde der eingangs genannten Art derart auszugestalten und weiterzubilden, dass eine besonders gute Erkennbarkeit oder Darstellbarkeit des Gebildes bei allen gängigen bildgebenden Verfahren mit konstruktiv einfachen Mitteln ermöglicht ist.

Erfindungsgemäß wird die voranstehende Aufgabe durch ein Gebilde mit den Merkmalen des Anspruchs 1 gelöst. Danach ist das Gebilde durch eine körperliche Ausprägung zur Begünstigung der visuellen Erkennbarkeit oder Erkennbarkeit bei bildgebenden Verfahren gekennzeichnet.

In erfindungsgemäßer Weise ist zunächst erkannt worden, dass durch geschickte Ausgestaltung eines bekannten Gebildes zur Markierung eines Orts oder Bereichs im oder am menschlichen oder tierischen Körper die voranstehende Aufgabe auf überraschend einfache Weise gelöst wird. In weiter erfindungsgemäßer Weise ist dann erkannt worden, dass die Realisierung einer körperlichen Ausprägung bei einem bekannten Gebilde die visuelle Erkennbarkeit oder Erkennbarkeit bei bildgebenden Verfahren ganz erheblich begünstigt. Obgleich bekannte Gebilde in Form von beispielsweise Fadenmarkierungen bislang durch bildgebende Verfahren nicht darstellbar waren, ist ein derartiges Gebilde mit der erfindungsgemäßen körperlichen Ausprägung durch sämtliche übliche bildgebenden Verfahren darstellbar. Auch die visuelle Erkennbarkeit mit beispielsweise bloßem Auge ist durch die erfindungsgemäße körperliche Ausprägung signifikant begünstigt.

Folglich ist mit dem erfindungsgemäßen Gebilde ein Gebilde angegeben, bei dem eine besonders gute Erkennbarkeit oder Darstellbarkeit des Gebildes bei allen gängigen bildgebenden Verfahren mit konstruktiv einfachen Mitteln ermöglicht ist.

Im Hinblick auf eine besonders gute Erkennbarkeit hat die Ausprägung einen schlauchartigen Hohlkörper oder kann einen kabelartigen Körper aufweisen der jedoch nicht Teil der Erfindung ist. Durch die Realisierung einer derartigen körperlichen Ausprägung in Form eines schlauchartigen Hohlkörpers oder kabelartigen Körpers wird das Gebilde bei allen gängigen bildgebenden Verfahren wesentlich besser erkennbar, als dies ohne die Ausprägung der Fall ist. Grundsätzlich kommen als bildgebende Verfahren insbesondere Ultraschall-, Röntgen-, MRT- und CT-Verfahren in Frage.

Zur weiteren Verbesserung der Erkennbarkeit hat der Hohlkörper mehrere, vorzugsweise in einer Reihe angeordnete und/oder durch Einschnürungen oder Einkerbungen gebildete Hohlräume. Derartige Hohlräume können vergleichbar einer Anordnung von Perlen auf einer Schnur hintereinander angeordnet sein. Dabei können die Hohlräume durch Einschnürungen oder geeignete Einkerbungen in dem schlauchartigen Hohlkörper gebildet sein. In derartigen Hohlräumen kann Luft oder ein definiertes Gas eingeschlossen sein, um ein Zusammendrücken der Hohlräume zu verhindern und die Hohlräume stabil aufrechtzuerhalten.

Zur weiteren Verbesserung der Erkennbarkeit des Gebildes kann der Hohlkörper kontrastgebende und/oder farbige Mittel aufweisen, die vorzugsweise in einem oder mehreren Hohlräumen angeordnet sind. Die Auswahl des Mittels kann vom jeweiligen bildgebenden Verfahren abhängig gemacht werden. Beispielsweise kann ein Mittel ausgewählt werden, das eine besonders gute Darstellbarkeit mit einem Ultraschall-Verfahren gewährleistet. Eine intraoperative Auffindbarkeit wird beispielsweise durch eine geeignete Farbauswahl des Mittels begünstigt. Beispielsweise könnte das Mittel blau sein, um einen besonders guten Kontrast zu umgebendem Gewebe wie Fett, Muskel oder Blut zu gewährleisten.

Ebenfalls im Hinblick auf eine weiter verbesserte Erkennbarkeit des Gebildes könnte ein kabelartiger Körper mehrere, vorzugsweise in einer Reihe angeordnete und/oder durch Einschnürungen oder Einkerbungen gebildete Abschnitte oder Teilelemente aufweisen. Ein derartiger kabelartiger Körper kann grundsätzlich als Vollmaterial ohne Hohlräume vorliegen.

Je nach Material des Gebildes kann der Hohlkörper oder der Körper zur Verbesserung der Erkennbarkeit mit kontrastgebenden und/oder farbigen Mitteln getränkt, benetzt oder eingefärbt sein. Falls das ausgewählte Material porös ist, kann der Hohlkörper oder der Körper in einfacher Weise mit kontrastgebenden und/oder farbigen Mitteln getränkt sein. Bei glatter oder geschlossener Oberflächenstruktur des Hohlkörpers oder Körpers ist eine Benetzung oder Einfärbung der Oberfläche mit kontrastgebenden und/oder farbigen Mitteln von Vorteil. Der Hohlkörper oder Körper kann jedoch auch mit dem kontrastgebenden und/oder farbigen Mittel eingefärbt sein, wobei eine "Durchfärbung" des Materials vorteilhaft ist.

Zum sicheren Einbringen oder Anordnen des Gebildes am gewünschten Ort oder im gewünschten Bereich kann das Gebilde, der Hohlkörper oder der Körper eine vorzugsweise integrierte, angegossene, angeschweißte oder angelötete Nadel aufweisen. Die Nadel kann je nach Anwendungsfalls aus Kunststoff oder Metall ausgebildet sein. Das Gebilde kann mit spezifisch hergestellten "Insertern" eingesetzt werden, z. B. Markierung nach Stanzbiopsie.

Zur weiteren Steigerung der Erkennbarkeit oder Darstellung des Gebildes kann das Gebilde, der Hohlkörper oder der Körper ringförmig oder dreidimensional am gewünschten Ort oder in dem gewünschten Bereich des Körpers anordenbar sein. Durch die Gestaltung insbesondere dreidimensionaler Strukturen mit dem erfindungsgemäßen Gebilde kann eine besonders gute Erkennbarkeit des Gebildes erreicht werden.

Das Gebilde, der Hohlkörper oder der Körper ist aus einem sich im menschlichen oder tierischen Körper auflösenden oder abbaubaren Material oder zur dauerhaften Markierung ausgebildet. Dabei kann das Material im Konkreten aus biologisch abbaubarem Material wie beispielsweise Chitin oder Chitosan bestehen oder eine dieser beiden Substanzen aufweisen. In vorteilhafter Weise besteht hierbei die Möglichkeit, die Resorptionsdauer des Materials durch eine spezielle Behandlung des Chitin oder Chitosan zu definieren. Damit kann der zeitliche Abbau im menschlichen oder tierischen Körper definiert werden. Beispielsweise kann das Gebilde zwei Jahre sichtbar sein und dann abgebaut werden.

Zur Anpassung an individuelle Gegebenheiten kann der Hohlkörper oder der Körper zwischen zwei Hohlräumen oder Abschnitten oder Teilelementen kürzbar sein. Dies kann mit einem geeigneten Schneidwerkzeug erfolgen. Damit kann das Gebilde in geeigneter Länge zum Einsatz kommen.

In weiter vorteilhafter Weise können die Hohlräume, Abschnitte oder Teilelemente zur Bereitstellung eines Maßstabs eine definierte Einzellänge aufweisen. Dabei können sämtliche an einem Gebilde ausgebildeten Hohlräume, Abschnitte oder Teilelemente die gleiche Länge oder auch unterschiedliche Einzellängen aufweisen. Beispielsweise kann jeweils ein Hohlraum, Abschnitt oder Teilelement 5mm lang sein.

Im Hinblick auf eine besonders komfortable und sichere Bereitstellung des Gebildes bei seinem medizinischen Einsatz kann das Gebilde in einer oder mehreren gewünschten Längen vorkonfektioniert sein. Zur individuellen Anpassbarkeit der Gebildelänge vor Ort kann das Gebilde als Endlosgebilde bereitgestellt sein. Auch eine Bereitstellung als Gebildepaket kann je nach Anwendungsfall vorteilhaft sein.

In weiter vorteilhafter Weise kann das Gebilde ein Faden sein.

Das erfindungsgemäße Gebilde kann in seiner Grundstruktur weich und flexibel sein, um an individuelle Anwendungsfälle anpassbar zu sein. Des Weiteren ist das erfindungsgemäße Gebilde durch alle bildgebenden Verfahren gut darstellbar, wobei es beispielsweise in Ringform, als 3D-Markierung oder auch als Einzelmarkierung einsetzbar ist.

Des Weiteren lässt es sich auf einfache Weise durch Punktion, Endoskopie oder intraoperativ am gewünschten Ort oder im gewünschten Bereich platzieren. Es kann gegebenenfalls im Körper verbleiben, um beispielsweise eine Metastasenkontrolle zu ermöglichen.

Das erfindungsgemäße Gebilde kann von einem Arzt oder anderem medizinischen Personal individuell - an den zu markierenden Ort oder Bereich angepasst - gekürzt werden. Eine derartige Kürzung kann insbesondere im Bereich von Einschnürungen oder Einkerbungen des Gebildes erfolgen.

Insgesamt ist mit dem erfindungsgemäßen Gebilde eine sehr gute intraoperative Darstellungsmöglichkeit inklusive einer leichten Auffindbarkeit ermöglicht. Nachträgliche Tumorsitzkontrollen sind zu 100% nachvollziehbar und dies auch nach Jahren. Der Anwendungsbereich des erfindungsgemäßen Gebildes ist sehr universell, wobei es sich insbesondere für strahlentherapeutische Anwendungen eignet. Beispielhafte Anwendungen sind außerdem die Tumorentfernung, die neoadjuvante Chemotherapie und eine Behandlung nach einer Operation. Häufig findet hierbei zunächst eine Strahlentherapie statt. Bis zur adjuvanten Chemotherapie dauert es dann manchmal bis zu einem Jahr. Des Weiteren ist das erfindungsgemäße Gebilde bei Tumorreduktionskontrollen durch Markierung der Außengrenzen einsetzbar.

Als Material für das erfindungsgemäße Gebilde bietet sich beispielsweise Goretex® an. Dieses Material ist in der Medizin für Fäden seit über 20 Jahren ohne Probleme im Einsatz, wobei dieses Material auch für dauerhafte Fäden verwendet werden kann. Auch andere textile Materialien oder Kunststoffe oder bioidentische Materialien können als Gebildematerial dienen.

In weiter vorteilhafter Weise kann das Gebilde an schon existierende Implantate und Körper, welche in den menschlichen oder tierischen Körper eingebracht oder an diesem angebracht werden oder worden sind, angebracht, angehängt oder eingewebt werden, beispielsweise an Netze, Implantate, Osteosynthesematerial etc.. Des Weiteren kann in vorteilhafter Weise aus der Struktur des Gebildes oder aus dem Gebilde ein neuer Körper hergestellt werden, beispielsweise Körper von Intrauterinpessaren IUP/IUD (Intrauterine Device). In einem solchen Fall kann in besonders vorteilhafter Weise der Sitz derartiger Pessare per Sonografie einfach dargestellt werden.

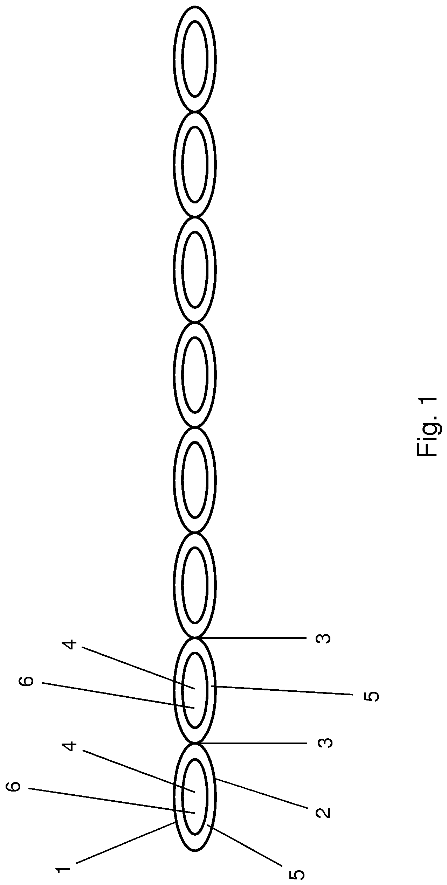

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt
- Fig. 1: in einer schematischen und geschnittenen Darstellung ein Ausführungsbeispiel eines erfindungsgemäßen Gebildes zur Markierung eines Orts oder Bereichs im oder am menschlichen oder tierischen Körper.

Fig. 1 zeigt in einer schematischen und geschnittenen Darstellung ein Ausführungsbeispiel eines erfindungsgemäßen Gebildes in Form eines Fadens zur Markierung eines Orts oder Bereichs im oder am menschlichen oder tierischen Körper. Im Konkreten zeigt Fig. 1 einen Längsschnitt durch den Faden, so dass ein Ende des Fadens links in der Fig. 1 und das andere Ende des Fadens rechts in der Fig. 1 gezeigt ist.

Im Hinblick auf eine besonders gute Erkennbarkeit oder Darstellbarkeit des Fadens bei allen gängigen bildgebenden Verfahren weist der erfindungsgemäße Faden eine körperliche Ausprägung 1 zur Begünstigung der visuellen Erkennbarkeit oder Erkennbarkeit bei bildgebenden Verfahren auf. Die körperliche Ausprägung 1 weist bei dem gezeigten Ausführungsbeispiel einen schlauchartigen Hohlkörper 2 auf. Der Hohlkörper 2 weist mehrere in einer Reihe angeordnete und durch Einschnürungen 3 oder Einkerbungen gebildete Hohlräume 4 auf. Fig. 1 zeigt somit einen Längsschnitt durch die jeweiligen Wände 5 der Hohlräume 4.

Im Hinblick auf eine besonders gute Darstellbarkeit des Fadens ist in den Hohlräumen 4 ein kontrastgebendes und/oder farbiges Mittel 6 angeordnet.

Die einzelnen Hohlräume 4 weisen eine definierte Einzellänge auf, so dass durch den Faden ein Maßstab bereitgestellt ist. Der Faden kann zwischen zwei Hohlräumen 4 durch beispielsweise ein Abschneiden auf einfache Weise gekürzt und somit an individuelle Erfordernisse angepasst werden.

Zusammengefasst ist der erfindungsgemäße Faden als schlauchartiger Hohlkörper 2 ausgebildet, der definiert abgegrenzte Hohlräume 4 aufweist.

Die Hohlräume 4 können beispielsweise jeweils 3-5mm lang sein und eine größte Breite von etwa 1mm aufweisen. Die Hohlräume 4 können weiterhin mit einem kontrastgebenden oder farbigen Mittel 6 gefüllt sein, so dass eine gute Darstellbarkeit bei insbesondere Ultraschall-Verfahren gewährleistet ist.

Des Weiteren kann der Faden mit kontrastgebenden und/oder farbigen Mitteln 6 getränkt oder benetzt sein, so dass eine gute Darstellbarkeit bei MRT-, CT- und Röntgen-Verfahren gewährleistet ist.

Des Weiteren kann der Faden mit einer Farbe, beispielsweise blau, eingefärbt sein, um eine gute Darstellbarkeit und intraoperative Auffindbarkeit zu gewährleisten. Dabei ist eine gute optische Abgrenzung zum umgebenden Gewebe wie Fett, Muskel, Blut usw. gegeben.

Eine Markersetzung oder Fadensetzung kann mit einem Clipsetzer direkt wie beispielsweise beim IUP-Legen erfolgen. Alternativ hierzu kann der Faden als einfacher chirurgischer Faden mit einer Nadel bewehrt sein, um intraoperative Markierungen zu setzten.

Ein vorkonfektionierter Faden kann beispielsweise eine Länge von bis zu 30cm aufweisen.

Des Weiteren kann das erfindungsgemäße Gebilde mit Materialien zur Verringerung von Kapselbildungen/ Abstoßungsreaktionen beschichtet werden, wie diese z.B. in der Netzchirurgie verwendet werden (z.B. Elevate PC®).

Hinsichtlich weiterer vorteilhafter Ausgestaltungen des erfindungsgemäßen Fadens wird zur Vermeidung von Wiederholungen auf den allgemeinen Teil der Beschreibung sowie auf die beigefügten Ansprüche verwiesen.

Schließlich sei ausdrücklich darauf hingewiesen, dass das voranstehend beschriebene Ausführungsbeispiel lediglich zur Erörterung der beanspruchten Lehre dient, diese jedoch nicht auf dieses Ausführungsbeispiel einschränkt.

### Bezugszeichenliste

- 1: Ausprägung
- 2: Hohlkörper
- 3: Einschnürung
- 4: Hohlraum
- 5: Wand
- 6: Mittel

## Patentansprüche

1. Formbares oder starres Gebilde zur Anordnung an einem Ort oder in einem Bereich und Markierung des Orts oder Bereichs im menschlichen oder tierischen Körper, mit einer körperlichen Ausprägung (1) zur Begünstigung der visuellen Erkennbarkeit oder Erkennbarkeit bei bildgebenden Verfahren, wobei die Ausprägung (1) einen schlauchartigen Hohlkörper (2) aufweist, **dadurch gekennzeichnet, dass** der Hohlkörper (2) mehrere durch Einschnürungen (3) oder Einkerbungen gebildete Hohlräume (4) aufweist, wobei das Gebilde oder der Hohlkörper (2) aus einem sich im menschlichen oder tierischen Körper auflösenden oder abbaubaren Material ausgebildet ist.

2. Gebilde nach Anspruch 1, **dadurch gekennzeichnet, dass** die Hohlräume (4) in einer Reihe angeordnet sind.

3. Gebilde nach Anspruch 1 oder 2, **dadurch gekennzeichnet, dass** der Hohlkörper (2), vorzugsweise in einem oder mehreren Hohlräumen (4), kontrastgebende und/oder farbige Mittel (6) aufweist.

4. Gebilde nach einem der Ansprüche 1 bis 3, **dadurch gekennzeichnet, dass** der Hohlkörper (2) mit kontrastgebenden und/oder farbigen Mitteln getränkt, benetzt oder eingefärbt ist.

5. Gebilde nach einem der Ansprüche 1 bis 4, **dadurch gekennzeichnet, dass** das Gebilde, der Hohlkörper (2) ringförmig oder dreidimensional anordenbar ist.

6. Gebilde nach einem der Ansprüche 1 bis 5, **dadurch gekennzeichnet, dass** der Hohlkörper (2) zwischen zwei Hohlräumen (4) oder Abschnitten oder Teilelementen kürzbar ist.

7. Gebilde nach einem der Ansprüche 1 bis 6, **dadurch gekennzeichnet, dass** die Hohlräume (4) zur Bereitstellung eines Maßstabs eine definierte Einzellänge aufweisen.

8. Gebilde nach einem der Ansprüche 1 bis 7, **dadurch gekennzeichnet, dass** das Gebilde in einer oder mehreren gewünschten Längen vorkonfektioniert oder als Endlosgebilde oder als Gebildepaket bereitgestellt ist.

9. Gebilde nach einem der Ansprüche 1 bis 8, **dadurch gekennzeichnet, dass** das Gebilde ein Faden ist.

## Claims

1. A shapeable or rigid structure for arrangement at a location or in an area and marking of the location or area in the human or animal body, having a physical manifestation (1) to promote visual recognizability or recognizability in imaging techniques, the manifestation (1) having a tubular hollow body (2), **characterized in that** the hollow body (2) has a plurality of cavities (4) formed by constrictions (3) or indentations, the structure or the hollow body (2) being formed from a material dissolvable or degradable in the human or animal body.

2. The structure according to claim 1, **characterized in that** the cavities (4) are arranged in a row.

3. The structure according to claim 1 or claim 2, **characterized in that** the hollow body (2) comprises contrasting and/or colored agents (6), preferably in one or more cavities (4).

4. The structure according to any one of claims 1 to 3, **characterized in that** the hollow body (2) is impregnated, wetted or dyed with contrasting and/or colored agents.

5. The structure according to any one of claims 1 to 4, **characterized in that** the hollow body (2) can be arranged in an annular or three-dimensional fashion.

6. The structure according to any one of claims 1 bis 5, **characterized in that** the hollow body (2) can be shortened between two cavities (4) or portions or subelements.

7. The structure according to any one of claims 1 to 6, **characterized in that** the cavities (4) have a defined individual length in order to provide a scale.

8. The structure according to any one of claims 1 to 7, **characterized in that** the structure is prefabricated in one or more desired lengths or provided as an endless structure or as a structure package.

9. The structure according to any one of claims 1 to 8, **characterized in that** the structure is a thread.

## Revendications

1. Structure déformable ou rigide destinée à être disposée dans un site ou dans une zone et à marquer ce site ou cette zone dans le corps humain ou animal, ayant une manifestation physique (1) pour favoriser l'identification visuelle ou l'identification dans le cadre de procédés d'imagerie, ladite manifestation (1) ayant un corps creux tubulaire (2), **caractérisée en ce que** ledit corps creux (2) a une pluralité de cavités (4) formées par des rétrécissements (3) ou des encoches, ladite structure ou ledit corps creux (2) étant réalisé en un matériau pouvant être dissous ou dégradés dans le corps humain ou animal.

2. Structure selon la revendication 1, **caractérisée en ce que** lesdites cavités (4) sont disposées en ligne.

3. Structure selon la revendication 1 ou la revendication 2, **caractérisée en ce que** ledit corps creux (2) comprend des agents contrastants et/ou colorés (6), de préférence dans une ou plusieurs cavités (4).

4. Structure selon l'une des revendications 1 à 3, **caractérisée en ce que** ledit corps creux (2) est imprégné, mouillé ou teinté avec des agents contrastants et/ou colorés.

5. Structure selon l'une des revendications 1 à 4, **caractérisée en ce que** ledit corps creux (2) peut être disposé de manière annulaire ou tridimensionnelle.

6. Structure selon l'une des revendications 1 à 5, **caractérisée en ce que** ledit corps creux (2) peut être raccourci entre deux cavités (4) ou tronçons ou sous-éléments.

7. Structure selon l'une des revendications 1 à 6, **caractérisée en ce que** lesdites cavités (4) ont une longueur individuelle définie afin de fournir une échelle.

8. Structure selon l'une des revendications 1 à 7, **caractérisée en ce que** ladite structure est préfabriquée dans une ou plusieurs longueurs souhaitées ou fournie comme une structure sans fin ou comme un ensemble de structures.

9. Structure selon l'une des revendications 1 à 8, **caractérisée en ce que** ladite structure est un fil.
